# EUROPEAN PATENT SPECIFICATION

(11) **EP 3 471 661 B1**
(45) Date of publication and mention of the grant of the patent: **23.04.2025**
(21) Application number: 17748918.4
(22) Date of filing: 21.06.2017
(51) Int. Cl.: A61F 2/12, A61F 2/00, A61L 27/30, C23C 14/06, C23C 14/35, C23C 14/50

(54) **MANUFACTURING PROCESS FOR A MEDICAL DEVICE COATED WITH A THIN CARBON FILM**
VERFAHREN ZUR HERSTELLUNG EINER MEDIZINISCHEN VORRICHTUNG MIT EINER DÜNNSCHICHTBESCHICHTUNG AUF DER BASIS VON KOHLENSTOFF
PROCÉDÉ DE PRODUCTION D'UN DISPOSITIF MÉDICAL REVÊTU D'UNE COUCHE MINCE À BASE DE CARBONE

(30) Priority: 21.06.2016 IT UA20164526
(43) Date of publication of application: 24.04.2019
(73) Proprietor: Humana Medical S.r.l., 35121 Padova (IT)
(72) Inventor: ORNAGHI, Oscar Marco, 35036 Montegrotto Terme (PD) (IT); BINOTTO, Radames, 35038 Torreglia (PD) (IT)
(74) Representative: Susanetto, Carlo
(86) International application number: PCT/IB2017/053698
(87) International publication number: WO 2017/221172

(56) References cited:
- EP-A2- 0 224 080
- EP-A2- 1 788 114
- WO-A1-2012/160070
- WO-A1-96/40446
- WO-A2-2009/016503
- IANNO N J ET AL: "DEPOSITION OF DIAMOND-LIKE CARBON ON A TITANIUM BIOMEDIAL ALLOY", THIN SOLID FILMS, ELSEVIER, AMSTERDAM, NL, vol. 270, no. 1/02, 1 December 1995 (1995-12-01), pages 275 - 278, XP000595232, ISSN: 0040-6090, DOI: 10.1016/0040-6090(95)06710-8

## Description

### Technical field

The present invention relates to a process for producing a medical device which is coated with a thin film of a biocompatible material based on carbon which is deposited on the medical device by means of a Physical Vapour Deposition process (abbreviated to PVD below, an acronym which is commonly used in the field).

Medical devices coated with a thin film based on carbon and which can be obtained by means of the above-mentioned process are also described.

### Technological background

In the field of production of medical devices, and in particular in the prostheses which are intended to be implanted in the human body for a greater or shorter period of time, there is known the need for maximizing the characteristics of biocompatibility of the medical device in order to reduce the possibility of undesirable negative developments of the post-operative recovery.

In fact, the introduction of a medical device, for example, a prosthesis, inside the human body is in general terms a very complex event for the organism and can unleash in the tissue surrounding the prosthesis a series of reactions, generally indicated as a foreign body reaction, which may produce the formation of scar tissue and painful inflammations. It is known, for example, that, when a prosthesis is inserted in soft tissue, thinking in particular of breast implants, the organism tends to react to the presence of the foreign body by forming a fibrous capsule of tissue around the prosthesis, sometimes having such dimensions and consistency as to alter the physical and mechanical characteristics of the prosthetic implant to an extent which compromises the functionality and which brings about pain, inflammation and infection which in some cases may require that the implant be removed.

Another example of a medical device which is intended to be implanted in the human body and to which the problems set out above apply is constituted by a net for containing internal organs.

This type of medical device, which is typically formed by interwoven filaments which are produced from polymer material, for example, on a polyolefin basis (optionally fluorinated) or polyester basis, is often used in reconstruction or reinforcement interventions of the abdominal wall, for example, in laparocele operations, where it is used to treat the intestine inside the abdominal cavity involved in the laparotomic injury.

In addition to all the biocompatibility problems mentioned above, this type of medical device has the additional requirement of maintaining over time the respective correct position inside the organism.

In order to comply with the above-mentioned biocompatibility requirement, there have been developed and improved medical devices which are coated with a layer of highly inert material from the biochemical point of view, the object of which is to prevent or at least limit to the greatest possible extent any biological interaction of the medical device with the surrounding natural organic tissue.

Naturally, this coating has to be able to ensure over time the absolute adhesion to the medical device under conditions in which it is intended to operate which in some cases may provide for loads and deformations which are increased and repeated over time.

A known solution provides for coating the medical device with a thin film based on carbon with a turbostratic structure.

This coating is obtained by means of a PVD technique which is known as sputtering (cathode sputtering), on the basis of which a beam of ions is accelerated against the surface of a target element formed by carbon. As a result of the bombardment with ions, carbon ions of the target element are projected into the surrounding environment, in which the medical device to be coated has been positioned beforehand. The carbon atoms deposited on the surface of the medical device combine with each other in a turbostratic type structure which is formed by a plurality of crystalline domains, each formed by parallel planes of carbon with nanometric dimensions. Therefore, each crystalline domain has an orientation of the planes which is different with respect to the orientation of the planes of the adjacent domains, producing a nanocrystalline structure in which there are present interstices having dimensions which are even smaller.

This particular structure confers on the thin carbon film chemico-physical characteristics which make it more similar to diamond than to graphite.

In fact, turbostratic carbon has optimum characteristics of adhesion, durability over time, biocompatibility and mechanical strength, which makes it particularly suitable for the use thereof as a coating of medical devices, in particular prostheses which are intended to be implanted in the human body.

Notwithstanding the optimum characteristics of the carbon coating obtained with the process mentioned above, however, there remains the requirement for increasing the biocompatibility of the prostheses so as to reduce the possibilities of foreign body reactions.

EP 1788114, EP 224080, WO 2009/016503, WO 2012/160070 and WO 96/40446 disclose examples of medical devices coated with a thin film of carbon and/or examples of processes for producing such devices according to the prior art.

### Disclosure of invention

The problem addressed by the present invention is to provide a medical device which is coated with a material based on carbon and a process for the production thereof which are structurally and functionally configured to exhibit improved biocompatibility with respect to known medical devices.

In the context of this problem, an object of the invention is to provide a process which does not substantially increase the production costs or complicate the production procedures.

This problem is solved and these objects are achieved by the present invention by means of a process in accordance with the appended claims.

In a first aspect thereof, the invention relates to a process for producing a medical device which is coated with a thin film based on carbon, according to claim 1.

Surprisingly, the Applicant has established that the prosthesis obtained with this process has better biocompatibility characteristics with respect to all the prostheses coated with a thin film of turbostratic carbon obtained with known processes.

In particular, the Applicant has found that the prosthesis obtained with the process of the invention releases over time nitrogen monoxide (NO), a compound whose important physiological function has been discovered in relatively recent times.

In fact, nitrogen monoxide has been found to be an important and effective transmitter of biological signals so as to induce vasodilation with a resultant increase in the blood flow.

At the same time, the nitrogen monoxide is capable of inhibiting the adhesion and aggregation of platelets in addition to inhibiting the expression of cytokines and adhesion molecules which attract inflammatory molecules. Therefore, the nitrogen monoxide which is released over time in the region of tissue being formed is capable of promoting therein the production of blood microvessels and therefore an appropriate vascularization.

Without wishing to link the validity of the present invention with a theoretical explanation of the phenomenon observed, the Applicant considers that during the formation of the thin film of carbon deposited on the surface of the medical device to be coated, in the interstices present between the nanocrystals which are orientated in a casual manner of the turbostratic carbon, there remain interpolated atoms of nitrogen and oxygen. These atoms become combined with each other so as to form nitrogen monoxide which is released gradually at the interface with the natural tissue.

In particular, the Applicant considers that the formation of nitrogen monoxide inside the thin film which is deposited on the medical device is strictly correlated with the quantity of nitrogen and oxygen which are residual in the processing chamber during the PVD step, that is to say, during the formation of the thin film of turbostratic carbon.

Consequently, the formation of nitrogen monoxide inside the turbostratic carbon being formed is strictly correlated with the residual pressure (that is to say, the degree of pressure reduction) obtained in the discharge step of the air inside the processing chamber which precedes the PVD step.

A residual pressure which is too high would compromise the characteristics of the thin film, in particular the adhesion thereof to the surface of the medical device, and even the formation of the turbostratic structure of the carbon, while a residual pressure which is too low would not leave a sufficient quantity of oxygen and nitrogen inside the processing chamber which can potentially be converted into nitrogen monoxide inside the turbostratic structure of the thin film of carbon.

Preferably, the residual pressure reached in the processing chamber during the discharge step which precedes the PVD step is approximately 3 x 10⁻⁴ Pa. The process for producing the medical device according to the invention provides for the medical device which is intended to be coated to be positioned inside a processing chamber.

The medical device can be produced from metal or from polymer material in accordance with the specific use thereof.

In a particularly preferred embodiment of the present invention, the medical device is a breast implant which is produced from polymer material based on silicone and which is intended for cosmetic surgery or reconstructive surgery. In an alternative embodiment, the medical device is a net for containing internal organs.

The medical device to be coated with the thin film based on turbostratic carbon is inserted into the processing chamber and positioned on suitable supports.

Preferably, these supports can be moved inside the processing chamber in such a manner that the medical device is directed towards the target element with portions of surface which are always different in the course of the PVD cycle so as to ensure that a uniform layer of thin film is deposited on the entire outer surface thereof.

Naturally, in accordance with the dimensions of the processing chamber and the medical devices to be coated, there can also be inserted a plurality of medical devices in the same PVD processing cycle.

In a preferred version, the PVD step is carried out by means of a Magnetron type apparatus in which the inert gas is ionized and accelerated by means of a variable electromagnetic field.

The inert gas used is preferably argon (Ar) which is introduced at a constant flow inside the processing chamber when the desired degree of pressure reduction is reached therein.

Preferably, following the introduction of the inert gas into the processing chamber, the pressure increases up to a value of approximately from 1 to 2 Pa. Preferably, the magnetic field is formed inside the processing chamber by a pair of electromagnets which have a nominal power from 1 to 5 KW and which are placed against a wall of the processing chamber and which are capable of generating a variable electromagnetic field which has a suitable intensity and geometry.

The magnetic field generated by the electromagnets inside the processing chamber causes the ionization of the inert gas present therein and the subsequent acceleration thereof towards the target element. There are expelled from the surface of the target element, which is bombarded by the group of ions, atoms of carbon which become deposited on the surface of the medical device (or the medical devices) directly exposed to the target element (sputtering technique).

Preferably, during the PVD step, the pressure inside the processing chamber is maintained between 0.01 Pa and 1 Pa.

Furthermore, during the PVD step, the supports on which the medical device(s) to be coated is/are mounted are moved inside the processing chamber so as to rotate about the different main axes thereof.

In this step, the temperature is preferably maintained between 20°C and 30°C. The PVD step is maintained for a predetermined time so as to deposit on the prosthesis a coating having a thickness between 100 and 1000 nm, preferably of approximately from 200 to 500 nm.

In a preferred embodiment, at the end of the PVD step, the pressure inside the processing chamber is brought to a level of pressure reduction between approximately 10⁻⁴ Pa and 10⁻³ Pa.

Subsequently, there is introduced into the processing chamber a suitable quantity of nitrogen (without oxygen) until the pressure is brought to between approximately 10⁻³ Pa and 10⁻² Pa.

In this manner, the oxygen is prevented from first coming into contact with the internal walls of the processing chamber and being able to be adsorbed at any active site thereof, thereby making it more difficult to reach in the processing chamber the degree of reduced pressure desired in the subsequent PVD cycle.

The pressure of the processing chamber is then brought up to the pressure conditions and atmospheric temperature conditions by means of the controlled introduction of air so that the medical device(s) can finally be removed from the processing chamber.

In a second aspect thereof, the invention relates to a medical device for containing internal organs comprising a net which is formed by a plurality of filiform elements which intersect at a respective plurality of nodes.

Preferably, the filiform elements are coated with a layer of turbostratic carbon while, in the region of at least some of the nodes, some portions of the filiform elements are not provided with the coating layer of turbostratic carbon.

The Applicant has found that a net for containing internal organs, in which the layer of turbostratic carbon is not distributed completely in each portion of the filiform elements which compose it but leaves some small portions without any covering at the nodes, promotes the maintenance over time of the position of the net inside the organism in which it has been implanted.

In fact, the presence of these small surface portions which are not coated promotes a small adhesion of natural tissue to the net which is thereby fixed in position.

The very reduced extent of these adhesion zones prevents the foreign body reaction from being particularly increased.

Furthermore, the fact that these zones without any coating of turbostratic carbon are located at some nodes and not necessarily at all the nodes allows an adhesion of the net distributed over locations in an almost uniform manner to be obtained.

Preferably, the net formed by the filiform elements has
meshes with a dimension of at least 1 mm.

### Brief description of the drawings

The characteristics and advantages of the invention will be better appreciated from the detailed description of a preferred embodiment thereof which is illustrated by way of non-limiting example with reference to the appended Figures, in which:
- Figure 1 is a schematic cross-section of an apparatus which is provided to operate according to the process of the present invention and
- Figure 2 is a schematic view of a medical device produced in accordance with a second aspect of the present invention.

### Preferred embodiments of the invention

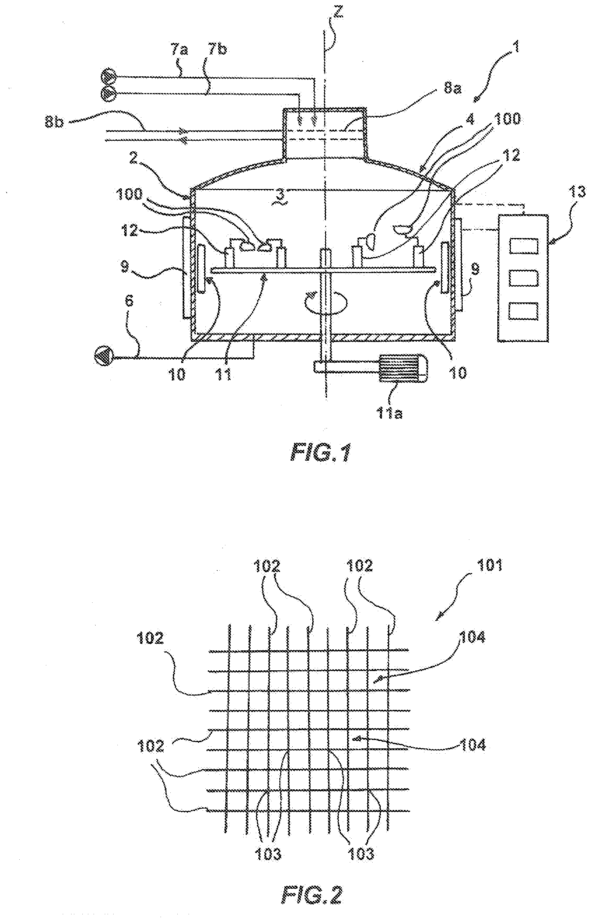

In Figure 1 there is generally designated 1 an apparatus which is provided to form a coating of a thin turbostratic carbon film on a medical device 100 in accordance with the process of the present invention.

The apparatus 1 has, in terms of the most general characteristics thereof, the functionality of a magnetron and is particularly provided to give rise to a Physical Vapour Deposition (PVD) processing operation by means of the sputtering technique.

To this end, the apparatus 1 comprises a container 2 which is of generally cylindrical form and which defines at the inner side thereof a processing chamber 3.

The container 2 is closed at the upper side by a cover 4 which can be lifted in order to afford access to the processing chamber 3.

The container 2 can be closed hermetically so that in the processing chamber 3 there can be achieved a desired level of reduced pressure by means of a dedicated pressure reduction line 6.

The container 2 is further connected to different gas sources which can be introduced inside the processing chamber 3. In particular there are provided an inert gas supply line 7a, for example, argon, a nitrogen supply line 7b.

At the inlet of the supply lines 7a and 7b in the processing chamber 3 there is further provided a vapour condensation device 8a which is provided to condense and eliminate any traces of compounds having a high molecular weight from the gases introduced into the processing chamber 3. To this end, the condensation device 8a is supplied by a line 8b in which a fluid flows at low temperature, for example, at -150°C.

The apparatus 1 further comprises one or more electromagnets 9 which are positioned externally behind a side wall of the container 2 and which are provided to generate a variable magnetic field inside the processing chamber 3 having a suitable intensity and geometry.

Inside the processing chamber 3, in the region of the electromagnets 9, there is positioned at least one target element 10 which is formed by carbon with a degree of purity of at least 99.99%.

The apparatus 1 further comprises a heating and/or cooling device which is capable of controlling the internal temperature of the processing chamber 3. There are further provided inside the processing chamber 3 supports 12 on which there can be fixed one or more medical devices 100 to be coated with a thin film of turbostratic carbon.

The supports 12 can be rotated about the main individual axes thereof and are mounted on a rotary table 11 which is caused to rotate about a vertical axis Z by a motor 11a. In this manner, during the PVD cycle the entire surface of the medical device 100 is exposed to the target element 10.

The apparatus 1 further comprises a control unit 13 which detects the operating parameters thereof, including the temperature and pressure inside the processing chamber 3, and controls the actuation thereof in respect of the individual mechanical and electrical components, in particular valves, pumps, motors and electromagnets.

The process for producing the medical device coated with a thin film of turbostratic carbon is carried out according to the following methods.

The medical devices 100 to be coated with the thin film of turbostratic carbon are positioned inside the processing chamber 3, where the target element 10 has previously been arranged, and they are fixed to the supports 12.

The medical devices 100, as set out above, may be breast implants which are produced from polymer based on silicone or in an alternative embodiment a net for containing internal organs, which is generally designated 101 in Figure 2.

The net 101 comprises a plurality of filiform elements 102 which intersect with each other at a respective plurality of nodes 103 so as to form a plurality of meshes 104. These meshes are preferably regular and have a characteristic dimension which is understood to be the minimum dimension of the section left open between the filiform elements which form the mesh of at least 1 mm, preferably of at least 3 mm.

Naturally, the medical devices 100 may be of any other form or type, including produced from metal material, such as, for example, bone prostheses or heart valves, which are intended to be implanted for a definite or indefinite time inside the human body.

Once the cover 4 is closed, the processing chamber 3 is placed at reduced pressure by means of the pressure reduction line 6 until reaching a residual pressure value between 10⁻⁴ Pa and 10⁻³ Pa, preferably of approximately 3 x 10⁻⁴ Pa.

At this point, there is introduced into the processing chamber 3 by means of the supply line 7a some inert gas, preferably argon, and the electromagnets 9 are actuated so as to generate inside the processing chamber 3 a variable magnetic field.

The power of the magnetic field, between 1 and 5 KW, is such that it ionizes the argon present in the processing chamber and accelerates the group of ions obtained thereby towards the target element 10.

The ion bombardment to which the target element 10 is subjected produces the rapid expulsion of carbon atoms from the surface of the target element 10, which are projected into the processing chamber 3 and in particular onto the exposed surface of the medical devices 100.

The carbon atoms are deposited on the surface of the medical devices and are aggregated with each other in a turbostratic structure forming a thin film which adheres powerfully to the medical devices 100.

These medical devices are moved continuously by the supports 12 so as to expose portions of surface of the medical devices 100 which are always different so that the layer of thin film being formed has a thickness which is substantially uniform and constant over the entire external surface of the medical device.

During this step, there is maintained inside the processing chamber 3 a pressure between 0.01 Pa and 1 Pa and a temperature between 20°C and 30°C.

The vapour deposition step of the carbon film lasts for a suitable time in accordance with the thickness of the coating of thin film desired between 100 nm and 1000 nm, preferably between 200 and 500 nm.

At the end of the PVD step, the pressure in the processing chamber 3 is again reduced as far as a value less than 10⁻³ Pa and then brought to a value of approximately 5 x 10⁻³ Pa by means of the introduction of a suitable quantity of nitrogen by means of the supply line 7b.

The processing chamber 3 is then vented in order to be brought to atmospheric pressure and opened to allow the removal of the medical devices 100, which are now suitably coated with a thin film of turbostratic carbon.

The medical devices which are coated with the process described above exhibit an extended release over time of nitrogen monoxide.

If the medical device comprises one or more nets 101, it is obtained that at the nodes 103 the filiform elements 102 are not provided with the coating of turbostratic carbon. This characteristic, which is reasonably caused by the mutual masking effect brought about by the filiform elements 102 which prevents the carbon atoms from becoming deposited on the portions of surfaces in which the filiform elements 102 are in contact with each other, advantageously allows the provision of superficial portions which are not coated, over a very reduced extent, less than 1 mm², in which it is possible that there will be produced an adhesion point of the net 101 with the surrounding natural tissue.

As a result of the reduced superficial extent of those adhesion points and the uniform distribution thereof over the entire surface extent of the net 101, there are produced the conditions for an effective action of maintaining the position of the net 101 inside the organism without bringing about relevant foreign body reactions.

It may be noted that this advantageous characteristic of the net 101 can also be obtained with known coating processes with turbostratic carbon, provided that they provide for the formation of the thin film of turbostratic carbon by means of PVD on a net which is already formed, that is to say, on a net in which the filiform elements are already interwoven with each other.

The present invention thereby solves the problem set out above with reference to the cited prior art.

## Claims

1. A process for producing a medical device (100) which is coated with a thin film based on carbon, comprising the steps of:
- positioning a medical device to be coated inside a processing chamber (3), in which there is provided a target element (10) of carbon;
- reducing the pressure in the processing chamber until reaching a residual pressure between 10⁻⁴ Pa and 10⁻³ Pa;
- introducing an inert gas in the processing chamber;
- starting in the processing chamber a physical vapour deposition step in which a group of ions of said inert gas is formed and is accelerated against the target element so as to cause the projection of carbon atoms from the target element towards the medical device;
- maintaining the physical vapour deposition step for a predefined time so as to deposit on the medical device a coating of a thin film of turbostratic carbon having a thickness between 100 nm and 1000 nm;
- venting the chamber and removing the coated medical device; and
**characterised in that**, at the end of the physical vapour deposition step and before the venting step, the pressure is brought to a value less than 10⁻³ Pa and there is subsequently introduced into the processing chamber a suitable quantity of nitrogen in order to bring the pressure to a value greater than 5 x 10⁻³ Pa.

2. A process according to claim 1, wherein the group of ions is accelerated against the target element by subjecting said inert gas to a variable electromagnetic field which has a power between 1 and 5 KW and inside which the target element is positioned.

3. A process according to claim 1 or claim 2, wherein, during the physical vapour deposition step, the pressure inside the processing chamber is maintained between 0.01 Pa and 1 Pa.

4. A process according to any one of the preceding claims, wherein the medical device is produced from metal material.

5. A process according to any one of claims 1 to 3, wherein the medical device is a breast implant which is produced from a silicone-based polymer material.

6. A process according to any one of claims 1 to 3, wherein the medical device comprises a plurality of filiform elements which are interwoven to form a net which is provided for containing internal organs.

## Patentansprüche

1. Verfahren zur Herstellung einer medizinischen Vorrichtung (100), die mit einem dünnen Film auf Kohlenstoffbasis beschichtet ist, wobei das Verfahren die Schritte aufweist:
- Positionieren einer zu beschichtenden medizinischen Vorrichtung in einer Bearbeitungskammer (3), in der ein Zielelement (10) aus Kohlenstoff vorgesehen ist;
- Reduzieren des Drucks in der Bearbeitungskammer, bis ein Restdruck zwischen 10⁻⁴ Pa und 10⁻³ Pa erreicht wird;
- Einführen eines Inertgases in die Bearbeitungskammer;
- Starten eines physikalischen Dampfabscheidungsschritts in der Bearbeitungskammer, bei dem eine Gruppe von Ionen des Inertgases gebildet und gegen das Zielelement beschleunigt wird, um die Projektion von Kohlenstoffatomen vom Zielelement in Richtung der medizinischen Vorrichtung zu bewirken;
- Aufrechterhalten des physikalischen Dampfabscheidungsschritts für eine vorgegebene Zeit, um auf der medizinischen Vorrichtung eine Beschichtung aus einem dünnen Film aus turbostratischem Kohlenstoff mit einer Dicke zwischen 100 nm und 1000 nm abzuscheiden;
- Entlüften der Kammer und Entfernen der beschichteten medizinischen Vorrichtung; und **dadurch gekennzeichnet, dass** am Ende des physikalischen Dampfabscheidungsschritts und vor dem Entlüftungsschritt der Druck auf einen Wert kleiner als 10⁻³ Pa gebracht wird und anschließend eine geeignete Menge Stickstoff in die Bearbeitungskammer eingeführt wird, um den Druck auf einen Wert größer als 5 x 10⁻³ Pa zu bringen.

2. Verfahren nach Anspruch 1, wobei die Ionengruppe gegen das Zielelement beschleunigt wird, indem das Inertgas einem veränderlichen elektromagnetischen Feld mit einer Leistung zwischen 1 und 5 kW ausgesetzt wird und innerhalb dessen das Zielelement positioniert ist.

3. Verfahren nach Anspruch 1 oder Anspruch 2, wobei während des physikalischen Dampfabscheidungsschritts der Druck innerhalb der Bearbeitungskammer zwischen 0,01 Pa und 1 Pa aufrechterhalten wird.

4. Verfahren nach einem der vorhergehenden Ansprüche, wobei die medizinische Vorrichtung aus metallischem Material hergestellt ist.

5. Verfahren nach einem der Ansprüche 1 bis 3, wobei die medizinische Vorrichtung ein Brustimplantat ist, das aus einem silikonbasierten Polymermaterial hergestellt ist.

6. Verfahren nach einem der Ansprüche 1 bis 3, wobei die medizinische Vorrichtung eine Mehrzahl fadenförmiger Elemente aufweist, die zu einem Netz verwoben sind, das zur Aufnahme innerer Organe vorgesehen ist.

## Revendications

1. Procédé de fabrication d'un dispositif médical (100) recouvert d'une couche mince à base de carbone, comprenant les étapes suivantes :
- positionner un dispositif médical à revêtir à l'intérieur d'une chambre de traitement (3), dans laquelle se trouve un élément cible (10) en carbone ;
- réduire la pression dans la chambre de traitement jusqu'à atteindre une pression résiduelle comprise entre 10⁻⁴ Pa et 10⁻³ Pa ;
- introduire un gaz inerte dans la chambre de traitement ;
- démarrer dans la chambre de traitement une étape de dépôt physique en phase vapeur au cours de laquelle un groupe d'ions dudit gaz inerte est formé et accéléré contre l'élément cible de manière à provoquer la projection d'atomes de carbone de l'élément cible vers le dispositif médical ;
- maintenir l'étape de dépôt physique en phase vapeur pendant une durée prédéfinie de manière à déposer sur le dispositif médical un revêtement constitué d'un film mince de carbone turbostratique d'une épaisseur comprise entre 100 nm et 1000 nm ;
- aérer la chambre et retirer le dispositif médical revêtu ; et **caractérisé en ce que**, à la fin de l'étape de dépôt physique en phase vapeur et avant l'étape d'aération, la pression est amenée à une valeur inférieure à 10⁻³ Pa et qu'une quantité appropriée d'azote est ensuite introduite dans la chambre de traitement afin d'amener la pression à une valeur supérieure à 5 x 10⁻³ Pa.

2. Procédé selon la revendication 1, dans lequel le groupe d'ions est accéléré contre l'élément cible en soumettant ledit gaz inerte à un champ électromagnétique variable d'une puissance comprise entre 1 et 5 KW, à l'intérieur duquel l'élément cible est positionné.

3. Procédé selon la revendication 1 ou la revendication 2, dans lequel, pendant l'étape de dépôt physique en phase vapeur, la pression à l'intérieur de la chambre de traitement est maintenue entre 0,01 Pa et 1 Pa.

4. Procédé selon l'une des revendications précédentes, dans lequel le dispositif médical est fabriqué à partir d'un matériau métallique.

5. Procédé selon l'une des revendications 1 à 3, dans lequel le dispositif médical est un implant mammaire fabriqué à partir d'un matériau polymère à base de silicone.

6. Procédé selon l'une des revendications 1 à 3, dans lequel le dispositif médical comprend plusieurs éléments filiformes qui sont entrelacés pour former un filet destiné à contenir des organes internes.
